(19) **Europäisches Patentamt / European Patent Office / Office européen des brevets**

(11) **EP 1 789 102 B1**

(12) **EUROPEAN PATENT SPECIFICATION**

(45) Date of publication and mention
of the grant of the patent:
**28.04.2010 Bulletin 2010/17**

(21) Application number: **04774927.0**

(22) Date of filing: **09.09.2004**

(51) Int Cl.:
**A61L 26/00** (2006.01)

(86) International application number:
**PCT/NL2004/000621**

(87) International publication number:
**WO 2006/028364 (16.03.2006 Gazette 2006/11)**

(54) **HYDROGEL WOUND COVERINGS**

HYDROGEL ZUR WUNDABDECKUNG

REVETEMENTS HYDROGELS POUR BLESSURES

(84) Designated Contracting States:
**AT BE BG CH CY CZ DE DK EE ES FI FR GB GR HU IE IT LI LU MC NL PL PT RO SE SI SK TR**

(43) Date of publication of application:
**30.05.2007 Bulletin 2007/22**

(73) Proprietor: **Broockeville Corporation N.V.
Willemstad,
Curaçao (AN)**

(72) Inventor: **OLEJNIK, A. K.
PL-93-590 Lodz (PL)**

(74) Representative: **Volmer, Johannes Cornelis et al
Exter Polak & Charlouis B.V.
P.O. Box 3241
2280 GE Rijswijk (NL)**

(56) References cited:
**US-A- 4 871 490      US-A- 5 540 033**

• **LUGAO A B ET AL: "Study of wound dressing structure and hydration/dehydration properties" RADIATION PHYSICS AND CHEMISTRY, ELSEVIER SCIENCE PUBLISHERS BV., AMSTERDAM, NL, vol. 52, no. 1-6, June 1998 (1998-06), pages 319-322, XP004492908 ISSN: 0969-806X**

• **HIGA O Z ET AL: "Biocompatibility study for PVP wound dressing obtained in different conditions" RADIATION PHYSICS AND CHEMISTRY, ELSEVIER SCIENCE PUBLISHERS BV., AMSTERDAM, NL, vol. 55, no. 5-6, 1 August 1999 (1999-08-01), pages 705-707, XP004172423 ISSN: 0969-806X**

• **MIRANDA L F ET AL: "Crosslinking and degradation of PVP hydrogels as a function of dose and PVP concentration" RADIATION PHYSICS AND CHEMISTRY, ELSEVIER SCIENCE PUBLISHERS BV., AMSTERDAM, NL, vol. 55, no. 5-6, 1 August 1999 (1999-08-01), pages 709-712, XP004172424 ISSN: 0969-806X**

• **LOPERGOLO L C ET AL: "Direct UV photocrosslinking of poly(N-vinyl-2-pyrrolidone) (PVP) to produce hydrogels" POLYMER, ELSEVIER SCIENCE PUBLISHERS B.V, GB, vol. 44, no. 20, September 2003 (2003-09), pages 6217-6222, XP004452539 ISSN: 0032-3861**

• **AJJI Z ET AL: "Production of hydrogel wound dressings using gamma radiation" April 2005 (2005-04), NUCLEAR INSTRUMENTS & METHODS IN PHYSICS RESEARCH, SECTION - B: BEAM INTERACTIONS WITH MATERIALS AND ATOMS, NORTH-HOLLAND PUBLISHING COMPANY. AMSTERDAM, NL, PAGE(S) 375-380 , XP004773471 ISSN: 0168-583X the whole document**

**Description**

The present invention relates to a method of preparing hydrogel wound coverings from polymers by irradiation cross-linking for use on animal bodies, in particular human bodies. The present invention also relates to a hydrogel wound covering thus prepared.

Such a method of manufacturing hydrogel wound coverings from synthetic and natural polymers by irradiation polymerisation is known in the art, for example from US-A-4 871 490 in the name of Rosiak et al.. This known method comprises the steps of pouring an aqueous solution of a synthetic polymer, for example polyvinylpyrrolidone (PVP), a natural polymer like agar, a so-called plasticizing agent such as polyethylene glycol (PEG) into a mould imparting a shape to the covering. The mould is closed, and then the composition contained in the mould is subjected to an irradiation dose of at least 25 kGy. According to this patent the aqueous solution has a composition of 2-20% by weight of synthetic polymers, not more than 5% by weight of natural polymers, not less than 75% by weight of distilled water and 1-3% by weight of plasticizing agent. A polyvinylpyrrolidone hydrogel according to this patent is currently marketed in Poland and some parts of central Europe under the trade name AQUA-GEL. The commercial formulation consists of 7% PVP, 1.5% polyethylene glycol and 1% agar. This formulation is said to be a good burn dressing, though its mechanical, swelling and drying properties leave something to be desired.

US-A-5 540 033 in the name of Fox et al. discloses a method of producing a sterile packaged adhesive hydrogel product by preparing an aqueous mixture of water, a polymer cross-linkable by radiation to form a hydrogel, and a cross-linking inhibitor in an amount sufficient to retard the cross-linking of the polymer when the mixture is exposed to radiation. Next the mixture thus prepared is shaped into a configuration, which is representative of the desired hydrogel product. Following shaping, the shaped mixture is enclosed in a sealed package, and subjected to a dose of radiation sufficient to simultaneously cross-link and sterilize the mixture in order to obtain the final hydrogel product. Preferred polymers used in this known method include polyethylene oxide, polyvinylpyrrolidone or mixtures thereof. A preferred cross-linking inhibitor is a food grade antioxidant like ascorbic acid. The polyvinylpyrrolidone is typically a polymer of N-vinyl-2-pyrrolidone having a weight average molecular weight of about 200,000-2,000,000 Dalton, advantageously around 1,000,000. The preferred concentration of polyvinylpyrrolidone in the aqueous mixture is about 10 to 35 weight %, most preferably 15 to 25 weight %. Additional additives can be included in the formulation. It is said that a humectant such as polyethylene glycol can be used to improve the physical properties of the hydrogel, in particular to extend the life-time thereof. In addition to the cross-linking inhibitor, a cross-linking promoter can be present. Ethylene glycol dimethacrylate is one of the examples mentioned of such cross-linking promoters. This known method can provide hydrogels having % gel values of at least about 80%. The absorptive capacity of the hydrogels, measured by the gel ratio, can be at least about 5.

US-A-5 578 661 in the name of Fox et al. discloses a gel-forming system for use in wound dressings. This know gel-forming system comprises an aqueous mixture of at least three polymeric components. The first polymeric component comprises a water-soluble polymer in an amount sufficient to increase the initial viscosity of the mixture and impart adhesive properties thereto. An example of such a water-soluble polymer is polyvinylpyrrolidone, which is advantageously present in an amount of between about 3 and 35% by weight of the gel-forming system. A hydrophilic water-soluble polymer mixture of polyvinylpyrrolidone and polyethylene oxide in a weight ratio of about 10/1 and 25/1 is preferred, wherein the polyvinylpyrrolidone is present in an amount in the range of about 10 to 25% by weight of the system. The second polymeric component is an acid-containing polymer, preferably a polymer of an acid or acid-forming compound or a copolymer of an acid of acid-forming compound and a monomer which forms a water-soluble compound. The third polymeric component is an amino-containing polymer, which is advantageously present in an amount in the range of 0.5 to 5% by weight. Among the preferred third components are heparin and agar. Additional additives like bactericides and antibiotics, can be present at a concentration of about 0.001 to about 3% by weight of the system. A humectant can be included in the gel-forming system in order to increase the solubility of the third or second component in the mixture. Polyethylene glycol is mentioned as one of the preferred humectants. Such a humectant may be present at a concentration of about 1 to 40%, preferably 5 to 20% by weight of the system. The examples presented in this patent are directed to formulations having a polyvinylpyrrolidone content of 10% of the mixture, wherein also polyethylene oxide is present. The gel ratio as a measure of the adsorption capacity of the hydrogels can exceed 5.

US-A-5 480 717 in the name of Kundel discloses processes for adhering a polymeric hydrogel to a substrate in order to obtain a hydrogel laminate with greatly improved delamination resistance. According to this patent a preferred hydrogel is cross-linked polyvinylpyrrolidone. The preferred polyvinylpyrrolidone polymers have a viscosity average molecular weight of about 150.000-450.000, and preferably about 200.000-300.000 Dalton. The hydrogel preferably comprises about 30 to 60, preferably about 40 to 50 weight % of the polymer complemented by about 40 to 70, preferably 50 to 60 weight % of water. It is said that if the molecular weight of the polyvinylpyrrolidone is too high it is not possible to make a solution with a high enough polyvinylpyrrolidone concentration, and as a result the adhesion to the polymeric adhesive layers after irradiation is not acceptable.

US-A-5 306 504 discloses adhesive hydrogel compositions based on a cross-linked polyvinylpyrrolidone. The adhesive hydrogel composition is prepared by mixing in an aqueous medium a water-soluble high molecular weight polyvinylpyrrolidone having ring opened pyrrolidone groups providing at least $1.5 \times 10^{-2}$ milliequivalents of carboxylic acid groups per gram of polymer, and a water-soluble multifunctional amine-containing polymer. The acid groups of the ring opened polyvinylpyrrolidone and the basic amine groups of the multifunctional amino-containing polymer are allowed to react in order to form a water-insoluble, water-swellable cross-linked ampholyte salt. The preparation takes place in an aqueous medium with a water content of about 40 to about 80% by weight. A plasticizer for tack development may be included in the reacting medium. An example of such a plasticizer is polyethylene glycol. The plasticizer may be present in the range of 20-55% by weight, based on the combined weight of the cross-linked polyvinylpyrrolidone and the plasticizer. Although the plasticizer may increase tack, it decreases gel strength.

US-A-4 646 730 in the name of Schonfeld et al. discloses the color stabilization of a polyvinylpyrrolidone based hydrogel dressing by the addition of magnesium trisilicate and optionally hydrogen peroxide and/or polyacrylate acid.

WO 03/034900 (EPICEPT CORPORATION) discloses an intradermal patch having a permeable backing, which is coated with a polyvinylpyrrolidone based hydrogel, containing one or more local anaesthetics. Preferably the hydrogel is a polyvinylpyrrolidone having an average molecular weight of about 500.000 to about 2.000.000 Dalton, more preferably about 900.000 Dalton to about 1.500.000 Dalton. The pre-gel hydrogel mixture, from which the patches according to this patent application are prepared, comprises a homogeneous mixture of about 5% to about 35% by weight, preferably about 10% to about 30%, more preferably about 15% to about 20% by weight of polyvinylpyrrolidone, and about 0,5% to about 20%, preferably about 2% to about 10% of a local anesthetic. The patches may comprise one or more additional components, like preservatives, stabilizers, absorptive agents, wound-healing agents, electrolyte and tonicity agents, viscosity-enhancing agents like agar, medicines, bioadhesive polymers, penetration enhancers and humectants.

The object of the present invention is to improve the physical properties of the hydrogel material in a wound covering. In particular the invention aims at improving the mechanical, swelling and drying properties thereof.

The method according to the invention of preparing hydrogel wound coverings from polymers by irradiation cross-linking comprises the steps of providing an initial aqueous solution comprising at least 15% of a synthetic polymer cross-linkable by irradiation, based on the weight of the mixture, at least one humectant, a natural polymer and water, pouring the initial aqueous solution into a mould for shaping, allowing the aqueous solution to mature in the mould during a period of time sufficient to obtain a semi-product having a content of at least 35% of synthetic polymer, removing the semi-product thus shaped from the mould, and subjecting the semi-product to irradiation in order to cross-link and sterilise the semi-product. In the method according the invention the aqueous solution comprises at least 15% of a synthetic polymer, which is cross-linkable by irradiation, based on the weight of the mixture. This amount of synthetic polymer is required to impart the required mechanical strength of the semi-product allowing further processing into the hydrogel product finally obtained. The preferred synthetic polymers cross-linkable by irradiation comprise polyvinylpyrrolidone, preferably having a weight average molecular weight of at least 300,000 Dalton.

Polyvinylpyrrolidone with such a high molecular weight does not dissolve in water homogeneously at concentrations over 30%. Therefore according to the invention it is necessary to prepare a more diluted starting solution and to allow the aqueous solution to mature in the mould during a period of time sufficient to obtain a homogeneous semi-product having a content of at least 35% of synthetic polymer. In addition to the synthetic polymer the aqueous solution comprises a humectant, preferably polyethylene glycol in order to improve the swelling properties of the hydrogel. The natural polymer used in the present invention is preferably agar, which is advantageously present in the range of 0.5-3.0% by weight in the solution. The aqueous solution can easily be prepared by mixing the original components in the required amounts into water. Thereafter the aqueous solution is poured into a mould in order to shape it into the desired configuration.

According to the invention the aqueous solution is allowed to mature in the mould during a period of time, that is sufficient to obtain a semi-product having at least a content of 35% of synthetic polymer. During this maturation period, water is partially liberated from the semi-product and evaporated, but most importantly the chains of the synthetic polymer, natural polymer and humectant undergo relaxation. By relaxation these components take thermodynamically preferred positions with the result that the concentrated components are homogenously distributed in water. After removal of the semi-product from the mould, the semi-product is subjected to irradiation in order to cross-link and sterilize the semi-product, e.g. by electron beams or γ-rays.

The duration of the maturation period is dependent from the initial concentration of the polymers and other components, the thickness of the layer in the mould, and the temperature at which the maturation occurs. Typically maturation can be carried out by allowing the composition to stand overnight at ambient temperature. Preferably the maturation period of time is at least 15 hours, at room temperature or slightly above.

In principle, other water-soluble synthetic polymers may be present in the initial aqueous solution in addition to polyvinylpyrrolidone, but their amount should be limited to a relatively small concentration such as 1-2%. The actual limit is dependent from the molecular weight of the respective polymer. The higher the molecular weight, the smaller the amount that will dissolve. Examples of such other water-soluble synthetic polymers comprise polyethyleneoxide (PEO), polyacrylamide (PAA), polyvinylalcohol (PVA) and polyacrylic acid. Polyethylene glycol is considered a humectant instead of a water-soluble synthetic polymer in the context of this specification.

The method according to the invention advantageously comprises an additional step of applying an adhesive plastic foil for adhesion to the skin onto the semi-product that has been removed from the mould. A preferred plastic foil is permeable to water vapour. Suitable adhesives and plastic foils are known in the art, e.g. from the patent literature cited above, and e.g. foils from 3M Company for medical purposes.

Advantageously the method also comprises the step of sealing the semi-product and adhesive plastic foil into a suitable packaging material prior to the irradiation treatment.

More preferably the final shape of the hydrogel coverings is obtained after maturation of the aqueous solution in the mould. In other words the semi-product is divided into articles having appropriate dimensions, e.g. cut into pieces, covered with adhesive plastic foil and then packed and sterilized.

Preferably the aqueous solution also comprises a cross-linking agent, because the presence of the humectant may decrease the gel fraction of the hydrogel wound covering. Preferably the amount of cross-linking agent is kept as low as possible, because the presence of the cross-linking may affect the transparency of the hydrogel. Additional additives may be present in the hydrogel wound covering according to the invention. Examples of suitable additives comprise preservatives, medicaments and antibacterial agents like allantonin. Allantonin is a medically acceptable antibacterial agent. It inhibits the growth of pathogenic microorganisms prior to irradiation of the semi-product.

The invention also relates to a hydrogel wound covering comprising a radiation cross-linked synthetic polymer in an amount of at least 35% by weight, a humectant and a natural polymer.

Preferably the covering comprises at least 2% by weight humectant, most preferably polyethylene glycol, and 0.5-3% by weight natural polymer such as agar.

The invention also relates to a packaged hydrogel wound dressing article comprising a hydrogel of a radiation cross-linked synthetic polymer in an amount of at least 35% by weight, a humectant and a natural polymer and an adhesive plastic foil, wrapped in a suitable packaging material.

A preferred aqueous solution used as starting material in the method according to the invention comprises:

20% polyvinylpyrrolidone (tradename: Kollidon K-90 (BASF))
10% polyethylene glycol (tradename: Lutrol 400 (BASF)
1% agar (available from Bristol Laboratories)
0-0.5% cross-linking agent, and
about 0.1% antibacterial agent
remainder (up to 100% distilled water).

After preparation of the semi-product and maturation during the night the preferred composition of the semi-product is about 35% polyvinylpyrrolidone, about 17% polyethylene glycol and 1.7% agar, while the concentration of the remaining components increases about 2 times with respect to the starting material.

After packaging the semi-product is sterilized with a dose of about 25 kGy.

Herein below the invention is illustrated in more detail, including reference to the attached drawing, wherein:

Fig. 1 shows the effect of a humectant on the swelling properties of a hydrogel;
Fig. 2 shows the effect of a humectant on the drying properties of a hydrogel;
Fig. 3 shows the effect of a cross-linking agent on the swelling properties of a hydrogel;
Fig. 4 shows the effect of a cross-linking agent on the percentage drying of PVP hydrogel;
Fig. 5 shows the percentage drying of a PVP hydrogel according to the invention versus a commercially available PVP hydrogel;
Fig. 6 shows the percentage swelling of a PVP hydrogel according to the invention versus a commercially available PVP hydrogel.

In the fig. "initial weight" means initial weight of ready hydrogel (after maturation and irradiation), and "initial solution" refers to the concentration of components in solution before maturation and irradiation.

The following properties of samples of the following examples were measured:

```
Percentage Swelling (%) = wt. swollen hydrogel - initial wt. hydrogel
x 100

                                initial wt. hydrogel
```

**EP 1 789 102 B1**

$$\text{Percentage Drying (\%)} = \frac{\text{wt. hydrogel}}{\text{initial wt. hydrogel}} \times 100$$

$$\text{Gel fraction} = \frac{\text{wt. dry gel}}{\text{wt. of PVP in semi-product}}$$

Different concentrations of PVP hydrogel (15%, 18%, 20% concentration of PVP in stock solution) were prepared using different amounts of PEG and agar. Irradiation of the polymeric mixtures was performed using a linear accelerator at an adsorbed dose of 25 kGy. The dose said was calibrated with a water calorimeter. Manual examination of the hydrogels thus prepared revealed that the 20% PVP mixture had the best mechanical properties.

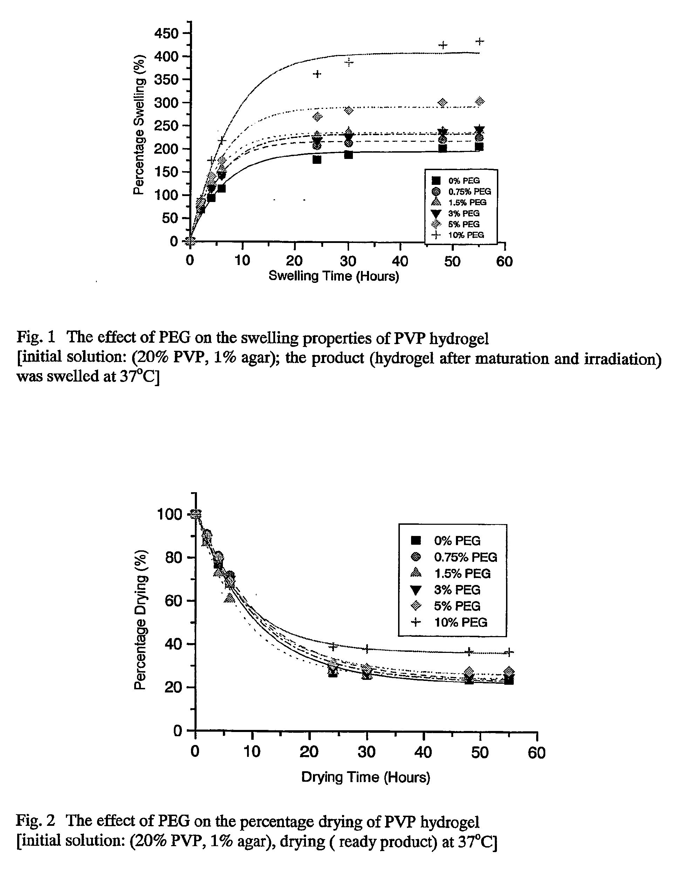

The swelling and drying properties of this hydrogel were tested using a balance with different amounts of PEG (0-10%) in the solution (before maturation). In general, addition of PEG can retard the drying process. Fig. 1 shows the effect of varying PEG concentration in the PVP hydrogel (20% PVP, 1% agar) on the swelling properties at 37° C. It is apparent that the percentage swelling increases with increasing PEG concentration. At 10% PEG, equilibrium swelling reaches a maximum of about 400%. Such a high equilibrium swelling is an indication of the adsorption capacity of the hydrogel wound covering. As shown in Table 1 below, gel fraction decreases to 0.58 at 10% PEG.

Table 1

| Gel fraction of PVP hydrogel (20% PVP, 1% agar) with different concentrations of PEG | |
|---|---|
| **% PEG** | **Gel fraction** |
| 0 | 0.90 |
| 0.75 | 0.90 |
| 1.5 | 0.90 |
| 3 | 0.88 |
| 5 | 0.94 |
| 10 | 0.58 |

Gel fraction can be related to cross-linking density, which in its turn can directly effect the mechanical properties of the hydrogel.

Fig. 2 shows the percentage drying of PVP hydrogel (20% PVP, 1% agar) at different concentrations of PEG. The drying patterns show similar curves for 0 to 5% PEG. Loss of water is 65% of the initial weight of the hydrogel after 55 hours. PVP having 10% PEG shows a better drying pattern with only 40% weight loss after the drying period.

Cross-linking agents can be used to improve the gel fraction. Polyethylene glycol dimethacrylate was studied in a PVP hydrogel formulation of 15% PVP and 1% agar at 10% PEG. Table 2 shows the results. The data obtained indicate that this cross-linking agent improves the cross-linking of this PVP hydrogel. The gel fraction increases with increasing amount of cross-linking agent.

Table 2

| Effect of polyethylene glycol dimethacrylate on the gel fraction of PVP hydrogel (15% PVP, 1% agar, 10% PEG) | |
|---|---|
| % Cross-linker | Gel fraction |
| 0 | 0.78 |
| 0.5 | 0.88 |
| 1 | 0.96 |
| 2 | 1.00 |

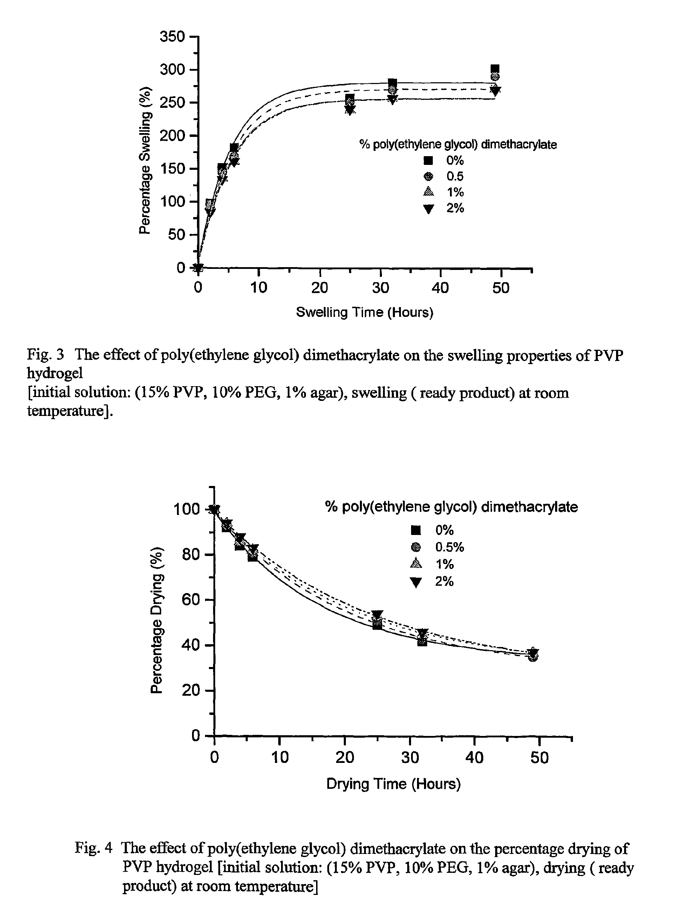

Fig. 3 shows the swelling pattern of this hydrogel. The percentage swelling decreases slightly with increasing amount of cross-linking agent. The amount of polyethylene glycol dimethacrylate does not affect the drying pattern of the PVP hydrogel. This is apparent from fig. 4. The curves indicate similar drying patterns. It was observed that the transparency of the hydrogel decreases as the amount of cross-linking agent increases.

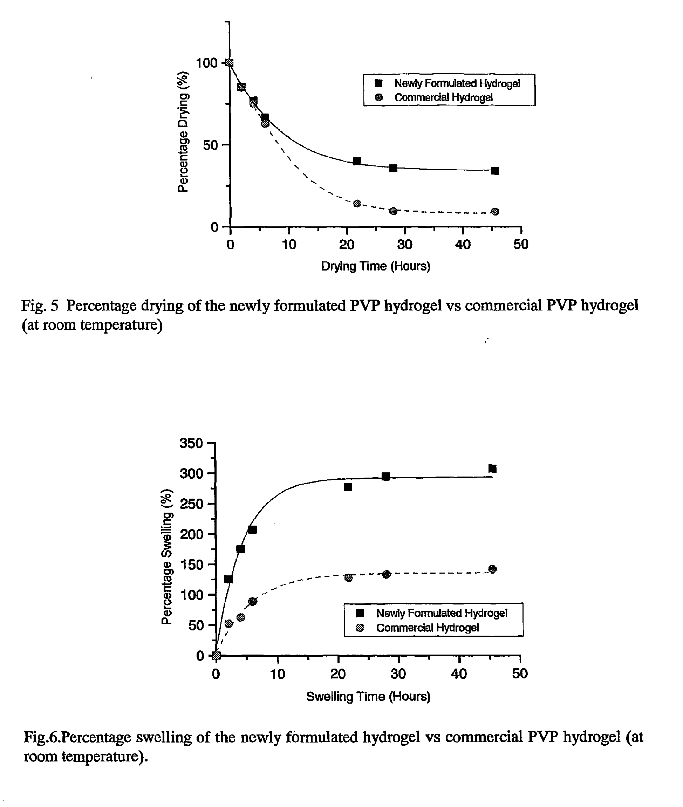

Fig. 5 and 6 show a comparison between a hydrogel according to the invention (15% PVP, 1% agar, 10% PEG, 0.1% cross-linking agent, 0.1% surfactant and 0.1% allantonin) and the commercially available AQUA GEL with regard to drying and swelling patterns.

## Claims

1. A method of preparing a hydrogel wound covering from polymers by irradiation cross-linking, comprising the steps of providing an initial aqueous solution comprising at least 15% of a synthetic polymer cross-linkable by irradiation, based on the weight of the mixture, at least one humectant, a natural polymer and water, pouring the aqueous solution into a mould for shaping,
   allowing the initial aqueous solution to mature in the mould during a period of time sufficient to obtain a semi-product having at least a content of 35 % of synthetic polymer,
   removing the semi-product thus shaped from the mould, and
   subjecting the semi-product to irradition in order to cross-link the semi-product.

2. A method according to claim 1, wherein the synthetic polymer cross-linkable by irradiation is polyvinylpyrrolidone.

3. A method according to claim 2, wherein the polyvinylpyrrolidone has a weight average molecular weight of at least 300,000 Dalton.

4. A method according to one of the preceding claims, wherein the humectant is polyethylene glycol.

5. A method according to claim 4, wherein the humectant is present in the initial aqueous solution in the range of 0.5-10 % by weight.

6. A method according to claim 4, wherein the content of humectant in the semi-product is at most 20 % by weight.

7. A method according to one of the preceding claims, wherein the natural polymer is agar.

8. A method according to one of the preceding claims, wherein the natural polymer is present in the range of 0.5-3.0 % by weight.

9. A method according to one of the preceding claims, wherein the initial aqueous solution also comprises a cross-linking agent.

10. A method according to one of the preceding claims, wherein the maturation period of time is at least 15 hours.

11. A. method according to one of the preceding claims, further comprising the step of applying an adhesive plastic foil for adhesion to the skin onto the semi-product that has been removed from the mould.

12. A method according to claim 11, further comprising the step of sealing the semi-product and adhesive plastic foil into a suitable packaging material prior to the irradiation treatment.

13. Hydrogel wound covering, obtainable by the process according to one of the preceding claims 1-12, comprising a radiation cross-linked synthetic polymer in an amount of at least 35% by weight, a humectant and a natural polymer.

## Patentansprüche

1. Verfahren zum Herstellen einer Hydrogel-Wundabdeckung aus Polymeren durch Vernetzung durch Bestrahlung, umfassend die folgenden Schritte:

   das Bereitstellen einer wässrigen Ausgangslösung umfassend wenigstens 15% eines durch Bestrahlung vernetzbaren synthetischen Polymers, bezogen auf das Gewicht der Mischung, wenigstens ein Netzmittel, ein natürliches Polymer und Wasser,

das Giessen der wässrigen Lösung in eine Form zur Formgebung,

das Reifenlassen der wässrigen Ausgangslösung in der Form während eines Zeitraums, der ausreicht, um ein Halbfabrikat zu erhalten, das wenigstens einen Gehalt von 35% an synthetischem Polymer aufweist,

das Entnehmen des so geformten Halbfabrikats aus der Form, und

das Aussetzen des Halbfabrikats einer Bestrahlung, um das Halbfabrikat zu vernetzen.

**2.** Verfahren nach Anspruch 1, wobei das durch Bestrahlung vernetzbare synthetische Polymer Polyvinylpyrrolidon ist.

**3.** Verfahren nach Anspruch 2, wobei das Polyvinylpyrrolidon ein gewichtsmittleres Molekulargewicht von wenigstens 300.000 Dalton aufweist.

**4.** Verfahren nach einem der vorangehenden Ansprüche, wobei das Netzmittel Polyethylenglycol ist.

**5.** Verfahren nach Anspruch 4, wobei das Netzmittel in der wässrigen Ausgangslösung im Bereich von 0,5 - 10 Gew.-% vorhanden ist.

**6.** Verfahren nach Anspruch 4, wobei der Gehalt an Netzmittel in dem Halbfabrikat höchstens 20 Gew.-% beträgt.

**7.** Verfahren nach einem der vorangehenden Ansprüche, wobei das natürliche Polymer Agar ist.

**8.** Verfahren nach einem der vorangehenden Ansprüche, wobei das natürliche Polymer im Bereich von 0,5 - 3,0 Gew.-% vorhanden ist.

**9.** Verfahren nach einem der vorangehenden Ansprüche, wobei die wässrige Ausgangslösung auch ein Vernetzungsmittel umfasst.

**10.** Verfahren nach einem der vorangehenden Ansprüche, wobei der Reifungszeitraum wenigstens 15 Stunden beträgt.

**11.** Verfahren nach einem der vorangehenden Ansprüche, außerdem umfassend den Schritt des Aufbringens einer Kunststoffklebefolie zur Adhäsion auf der Haut auf das Halbfabrikat, welches aus der Form entnommen worden ist.

**12.** Verfahren nach Anspruch 11, außerdem umfassend den Schritt des Einschließens bzw. Versiegelns des Halbfabrikats und der Kunststoffklebefolie in einem geeigneten Verpackungsmaterial vor der Bestrahlungsbehandlung.

**13.** Hydrogel-Wundabdeckung, erhältlich durch das Verfahren nach einem der vorangehenden Ansprüche 1 - 12, umfassend ein durch Bestrahlung vernetztes synthetisches Polymer in einer Menge von wenigstens 35 Gew.-%, ein Netzmittel und ein natürliches Polymer.

**Revendications**

**1.** Procédé de préparation d'un revêtement d'hydrogel pour plaies à partir de polymères en effectuant une réticulation par irradiation, ledit procédé comprenant les étapes consistant à

fournir une solution aqueuse initiale comportant au moins 15% d'un polymère de synthèse réticulable par irradiation, rapportés au poids du mélange, au moins un agent humidifiant, un polymère naturel et de l'eau,

verser la solution aqueuse dans un moule de mise en forme,

laisser la solution aqueuse initiale venir à maturation dans le moule pendant un laps de temps suffisant pour obtenir un demi-produit ayant une teneur en polymère de synthèse d'au moins 35%,

retirer du moule le demi-produit ainsi mis en forme, et

soumettre le demi-produit à une irradiation afin de réticuler le demi-produit.

**2.** Procédé selon la revendication 1, dans lequel le polymère de synthèse réticulable par irradiation est de la polyvinylpyrrolidone.

**3.** Procédé selon la revendication 2, dans lequel la polyvinylpyrrolidone a un poids moléculaire moyen d'au moins 300 000 Daltons.

**4.** Procédé selon l'une des revendications précédentes, dans lequel l'agent humidifiant est du polyéthylène glycol.

**5.** Procédé selon la revendication 4, dans lequel l'agent humidifiant est présent dans la solution aqueuse initiale dans la gamme de 0,5 à 10% en poids.

**6.** Procédé selon la revendication 4, dans lequel la teneur en agent humidifiant dans le demi-produit est au maximum de 20% en poids.

**7.** Procédé selon l'une des revendications précédentes, dans lequel le polymère naturel est de l'agar-agar.

**8.** Procédé selon l'une des revendications précédentes, dans lequel le polymère naturel est présent dans la gamme de 0,5 à 3,0% en poids.

**9.** Procédé selon l'une des revendications précédentes, dans lequel la solution aqueuse initiale comprend également un agent réticulant.

**10.** Procédé selon l'une des revendications précédentes, dans lequel la période de maturation est d'au moins 15 heures.

**11.** Procédé selon l'une des revendications précédentes, comprenant en outre l'étape consistant à appliquer sur le demi-produit, qui a été retiré du moule, une feuille de matière plastique adhésive destinée à adhérer à la peau.

**12.** Procédé selon la revendication 11, comprenant en outre l'étape consistant à enfermer hermétiquement le demi-produit et la feuille de matière plastique adhésive dans un matériau d'emballage approprié avant le traitement par irradiation.

**13.** Revêtement d'hydrogel pour plaies, pouvant être obtenu par le procédé selon l'une des revendications précédentes 1 à 12, ledit revêtement comprenant un polymère de synthèse réticulé par irradiation dans une quantité d'au moins 30% en poids, un agent humidifiant et un polymère naturel.

Fig. 1 The effect of PEG on the swelling properties of PVP hydrogel
[initial solution: (20% PVP, 1% agar); the product (hydrogel after maturation and irradiation) was swelled at 37°C]

Fig. 2 The effect of PEG on the percentage drying of PVP hydrogel
[initial solution: (20% PVP, 1% agar), drying ( ready product) at 37°C]

Fig. 3 The effect of poly(ethylene glycol) dimethacrylate on the swelling properties of PVP hydrogel
[initial solution: (15% PVP, 10% PEG, 1% agar), swelling ( ready product) at room temperature].

Fig. 4 The effect of poly(ethylene glycol) dimethacrylate on the percentage drying of PVP hydrogel [initial solution: (15% PVP, 10% PEG, 1% agar), drying ( ready product) at room temperature]

Fig. 5 Percentage drying of the newly formulated PVP hydrogel vs commercial PVP hydrogel (at room temperature)

Fig.6.Percentage swelling of the newly formulated hydrogel vs commercial PVP hydrogel (at room temperature).

**EP 1 789 102 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4871490 A
- US 5540033 A, Fox
- US 5578661 A, Fox
- US 5480717 A
- US 5306504 A
- US 4646730 A, Schonfeld
- WO 03034900 A